# EUROPÄISCHE PATENTSCHRIFT

(11) **EP 3 359 120 B1**
(45) Veröffentlichungstag und Bekanntmachung des Hinweises auf die Patenterteilung: **11.12.2019**
(21) Anmeldenummer: 16781309.6
(22) Anmeldetag: 30.09.2016
(51) Int. Cl.: A61Q 17/00, A61Q 19/00, A61K 8/34, A61K 8/42

(54) **STABILE LÖSUNG VON HEXAMIDIN-SALZEN IN ALKANDIOL-WASSER-GEMISCHEN MIT ANTI-MIKROBIELLER UND HAUTBEFEUCHTENDER WIRKUNG**
STABLE SOLUTION OF SALTS OF HEXAMIDINE IN MIXTURES OF ALKANEDIOL AND WATER WITH ANTIMICROBIAL AND SKIN MOISTURING ACTION
SOLUTION STABLE DES SELS DE HEXAMIDINE DANS DES MELANGES D'ALCANEDIOL ET D'EAU AVEC ACITIVITÉ ANTIMICROBIELLE ET HUMIDIFIÉ DE LA PEAU

(30) Priorität: 05.10.2015 DE 102015116835
(43) Veröffentlichungstag der Anmeldung: 15.08.2018
(73) Patentinhaber: Minasolve SAS, 59310 Beuvry-la-Forêt (FR)
(72) Erfinder: NAHRWOLD, Markus, 32423 Minden (DE); KONATÉ, Nadia, 39108 Magdeburg (DE)
(74) Vertreter: Brantsandpatents bvba
(86) Internationale Anmeldenummer: PCT/EP2016/073475
(87) Internationale Veröffentlichungsnummer: WO 2017/060173

(56) Entgegenhaltungen:
- EP-A1- 1 477 157
- EP-A1- 1 779 839
- WO-A2-2012/170695
- DE-U1- 20 221 386

## Beschreibung

Die Erfindung betrifft chemisch und physikalisch stabile Lösungen von Hexamidin-Salzen in einer Mischung aus Wasser und mindestens einem Alkandiol. Das mindestens eine Alkandiol weist eine gerade oder verzweigte C3- bis C5-Kohlenstoffkette auf. Der ClogP beträgt -0,2 bis -1,1. Zur chemischen Stabilisierung wird der pH-Wert der erfindungsgemäßen Lösungen im Bereich 3,0 bis 6,0 eingestellt. Die erfindungsgemäßen Lösungen zeigen synergistische antimikrobielle und hautbefeuchtende Wirkungen, welche die Wirkungen ihrer Einzelkomponenten übertreffen.

Körperpflegemittel und Kosmetika werden alltäglich in einer nicht-sterilen Umgebung verwendet. Als wasserbasierte Gemische bieten sie einen guten Nährboden für gesundheitsschädliche Mikroorganismen. Erst der Zusatz antimikrobieller Wirkstoffe gewährleistet eine ausreichende Konservierung der Produkte, und garantiert damit die Sicherheit der Verbraucher. Viele der in Körperpflegeprodukten eingesetzten Konservierungsmittel können jedoch neben ihren erwünschten schützenden Eigenschaften auch unerwünschte Nebenwirkungen zeigen. So zeigen die als "Parabene" bekannten 4-Hydroxybenzoesäurealkylester eine schwache hormonelle Wirkung. Isothiazolinon-Derivate sowie antimikrobielle Duftstoffe wie z.B. Benzylalkohol oder Farnesol, erwiesen sich als sensibilisierend bzw. allergieauslösend. Die Verwendung Formaldehyd-abspaltender Substanzen wird aufgrund der krebserzeugenden Wirkung des Formaldehyds zunehmend als fragwürdig angesehen. Auch halogenhaltige Konservierungsmittel wie Chlorphenesin, Triclosan oder Bronopol sind aufgrund ihrer Giftigkeit und möglicher sensibilisierender Eigenschaften in die Kritik geraten. Vor diesem Hintergrund ist die effektive und sichere Konservierung von Körperpflegeprodukten eine Herausforderung für alle Hersteller derartiger Artikel. Dies betrifft besonders Produkte für Menschen mit empfindlicher oder gereizter Haut, sowie Gesichts- und Intimpflegeprodukte und Produkte für Kinder.

Im Vergleich mit den zuvor genannten Konservierungsmitteln ist Hexamidin-Diisethionat (I) (chemischer Name: 2-Hydroxyethansulfonsäure-4,4'-[1,6-hexandiylbis(oxy)]dibenzolcarbox-imidamid, CAS-Nr. 659-40-5) ein sehr mildes und sicheres antimikrobielles Mittel mit guter Verträglichkeit und geringem Risiko einer Hautirritation (International Journal of Toxicology 2007, vol. 26, Suppl. 3, S. 79-88). Hexamidin-Diisethionat zeigt ein breites antimikrobielles Wirkspektrum gegenüber Bakterien, Hefen und Pilzen. Die Substanz wird unter anderem als antiseptisches Mittel in der Mund- und Augenpflege eingesetzt. Als Feststoff ist Hexamidin-Diisethionat jedoch nur schwer handhabbar. In Pulverform kann es bei der Handhabung Staub entwickeln, der die Atemwege reizt. In Wasser sind Hexamidin-Salze nur mäßig löslich, weshalb sie unter Erwärmen aufgelöst werden müssen. Darüber hinaus kann das kationische Hexamidin in Lösung mit anionischen Komponenten schwerlösliche Salze bilden. Bei hohen pH-Werten ist Hexamidin generell schwer löslich in Wasser. All dies kann den Einsatz von Hexamidin-Salzen in kosmetischen oder dermatologischen Produkten erschweren. Als alleiniges Konservierungsmittel ist Hexamidin-Diisethionat zudem nicht in jedem Fall ausreichend effektiv. Wünschenswert wäre daher ein stabiles flüssiges Konzentrat von Hexamidin in einem Lösemittel, das seine konservierende Wirkung verstärkt und dabei gleichzeitig die Handhabung vereinfacht.

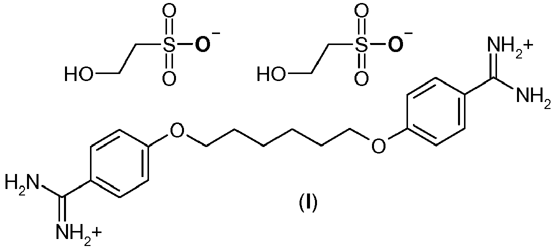

Eine alternative Möglichkeit zum Schutz von Kosmetika vor mikrobiologischer Kontamination stellt der Einsatz von Alkan-Polyolen mit antimikrobieller Wirkung dar. Dabei finden vor allem langkettige C6- bis C10-Alkandiole wie 1,2-Hexandiol, 1,2-Octandiol und 1,2-Decandiol Anwendung. Aufgrund ihrer hautirritierenden Wirkung, ihres charakteristischen Geruchs, ihrer geringen Wasserlöslichkeit und ihrer destabilisierenden Wirkung auf Emulsionen und Gele sind diese Substanzen jedoch nur in begrenzter Konzentration einsetzbar. Weiterhin erzeugen sie ein fettiges Hautgefühl, das nicht immer erwünscht ist. Demgegenüber weisen kurzkettige Diole mit C3- bis C5-Alkylketten eine höhere Hautkompatibilität auf. Diese Diole sind in der Regel geruchlos, wasserlöslich und weitgehend kompatibel mit O/W-Emulsionen und anderen galenischen Formen. Zudem erzeugen C3- bis C5-Alkandiole ein angenehmes und neutraleres Hautgefühl als die langkettigen Diole. Allerdings ist ihre antimikrobielle Aktivität deutlich geringer ausgeprägt, als die der langkettigen Diole (siehe Symrise, DE20221386U1, Anmeldedatum 17.10.2002). Für eine kosteneffektive Konservierung kosmetischer Produkte werden die kurzkettigen C3- bis C5-Diole daher in der Regel mit weiteren Konservierungsmitteln kombiniert. Weiterhin sind kurzkettige Diole als Hautpflegemittel und Feuchtigkeitsspender bekannt (siehe u.a. Symrise, US20100216892A1, Priorität 07.06.2007). Hydrophilere C3-Polyole wie 1,2-Propandiol oder Glycerin werden in der Regel in größeren Mengen zur Hautbefeuchtung eingesetzt. Dabei können sie jedoch ein unangenehm klebriges Hautgefühl erzeugen. Insbesondere 1,2-Propandiol kann zudem hautirritierend wirken. Wünschenswert wäre daher eine Kombination mittelkettiger Alkandiole mit konservierenden Substanzen wie Hexamidin-Diisethionat. Idealerweise würde durch diese Kombination sowohl die antimikrobielle Wirkung, als auch die hautpflegende Wirkung verstärkt werden.

In der Literatur sind Kombinationen aus Alkandiolen und Hexamidin-Diisethionat beschrieben:
EP1477157B1 (Procter & Gamble, Priorität: 16.05.2003) offenbart Mischungen, die C6- bis C9-Alkandiole, Amidine und Wasser enthalten. Dabei wird eine stabilisierende Wirkung der C6- bis C9-Diole auf Amidine in wässriger Lösung offenbart, wobei eine Abhängigkeit der Stabilisierung von dem negativen dekadischen Logarithmus des Wasser/n-Octanol-Verteilungskoeffizienten ClogP (auch bekannt als "log KOW") festgestellt wird. Gemäß der Patentschrift weisen ausschließlich Alkandiole mit einem ClogP-Wert von +0,2 bis +1,55 eine stabilisierende Wirkung auf Amidine in wässriger Lösung auf. Als besonders geeignet werden C6- bis C9-Alkandiole wie 1,2-Hexandiol (ClogP = +0,25), 1,7-Heptandiol (ClogP = +0,46) oder 1,2-Octandiol (ClogP = +1,32) genannt. Weiterhin wird offenbart, dass hydrophilere Alkandiole mit ClogP < 0,2, wie auch hydrophobere Alkandiole mit ClogP > 1,55 keine stabilisierende Wirkung auf Amidine in Lösung haben. Als geeigneter pH-Wert-Bereich für Amidine wird pH 4 bis 7,5, bevorzugt pH 5 bis 6,5 angegeben, da pH-Werte oberhalb 7,5 zu einer basischen Hydrolyse des Amidins führen.

Hexamidin-Diisethionat (I) ist kommerziell erhältlich, unter anderem von der Firma BASF unter dem Markennamen "Elestab HP100". In dem dazugehörigen Datenblatt wird ein kompatibler pH-Wert-Bereich von 3-8 angegeben. Ferner wird erwähnt, dass Hexamidin-Diisethionat in 1,2-Propandiol löslich ist. Von der Firma COBIOSA ist unter dem Markennamen COBIOSTAB 400 eine Lösung erhältlich, die aus 1,2-Propandiol, Wasser und Hexamidin-Diisethionat zusammengesetzt ist. Nachteilig an 1,2-Propandiol als Lösemittel ist seine eingeschränkte Mischbarkeit mit lipophilen Phasen, seine geringe eigenständige antimikrobielle Aktivität und die erwähnte hautirritierende Wirkung.

Aufgabe der Erfindung ist die Schaffung leicht handhabbarer Lösungen von Hexamidin-Salzen in C3- bis C5-Alkandiolen und Wasser. Die Lösungen sollen synergistische antimikrobielle und Haut befeuchtende Wirkungen zeigen und in einem weiten Temperaturbereich chemisch und physikalisch stabil sein.

Erfindungsgemäß wird diese Aufgabe gelöst durch eine temperaturstabile Lösung bestehend aus
a) 0,1 - 10 Gew.-% mindestens eines Hexamidin-Salzes,
b) 40 - 95 Gew.-% eines Alkandiols mit einer C3- bis C5-Kohlenstoffkette und einem ClogP von -0,2 bis -1,1,
c) 5 - 60 Gew.-% Wasser
   und
d) einem Additiv oder mehreren Additiva zur Einstellung des pH-Wertes der Lösung auf 3,0 bis einschließlich 6,0,
wobei die Gesamtmenge der Komponenten a) bis d) 100 Gew.-% beträgt.

Vorzugsweise ist das Hexamidin-Salz a) Hexamidin-Diisethionat.
Das mindestens eine Alkandiol b) ist ausgewählt unter 1,3-Propandiol, 1,2-Butandiol, 1,3-Butandiol (Butylenglycol), 2,3-Butandiol, 1,2-Pentandiol (Pentylenglycol), 1,5-Pentandiol und 3-Methyl-1,3-butandiol (Isopentyldiol), vorzugweise 1,2-Pentandiol und 1,3-Butandiol, insbesondere bevorzugt 1,2-Pentandiol.

Erwartungsgemäß sind kurzkettige C3- bis C5-Alkandiole schlechte Lösemittel für Hexamidin-Diisethionat (siehe Tabelle 1, Vergleichsbeispiele L-Q). Überraschenderweise gilt dies auch für das kurzkettige 1,3-Propandiol (Vergleichsbeispiel R), während das isomere 1,2-Propandiol ein gutes Lösemittel für Hexamidin-Diisethionat darstellt (Vergleichsbeispiel S). Auch reines Wasser löst Hexamidin-Diisethionat bei 20 °C lediglich bis zu einer Sättigungskonzentration von 3,5 % (Vergleichsbeispiel T). Überraschend wurde jedoch gefunden, dass Hexamidin-Diisethionat in Mischungen aus Wasser und kurzkettigen C4- bis C5-Alkandiolen bzw. 1,3-Propandiol sehr gut löslich ist. Diese Beobachtung steht im Gegensatz zur Literatur. Als besonders geeignete Lösemittel erwiesen sich Kombinationen von Wasser und Alkandiolen mit einem ClogP-Wert von -0,2 bis -1,1 (siehe Tabelle 1, Beispiele A-K). Die angewendete Methode zur Ermittlung des Aggregatzustandes der Mischungen wird in Beispiel 1 detailliert beschrieben.

**Tabelle 1: Aggregatzustände von Mischungen aus Hexamidine Diisethionate, Alkandiolen und Wasser (- = Feststoff, 0 = zweiphasige Mischung, + = klare Flüssigkeit)**

| **#** | **Alkandiol** | | | **Hexamidine Diisethionat** | **H₂O** | **Aggregatzustände der Mischungen bei:** | | | | | |
|---|---|---|---|---|---|---|---|---|---|---|---|
| | **Substanz** | **ClogP** | **%** | | | **-20°C** | **-10°C** | **0 °C** | **+10°C** | **+20°C** | **+50°C** |
| **A** | 1,2-Pentandiol | -0,28 | 40 % | 5 % | 55 % | 0 | 0 | + | + | + | + |
| **B** | 1,2-Pentandiol | -0,28 | 70 % | 5 % | 25 % | 0 | + | + | + | + | + |
| **C** | 1,2-Pentandiol | -0,28 | 75 % | 5 % | 20 % | + | + | + | + | + | + |
| **D** | 1,2-Pentandiol | -0,28 | 85 % | 5 % | 10 % | + | + | + | + | + | + |
| **E** | 1,2-Pentandiol | -0,28 | 90 % | 5 % | 5 % | + | + | + | + | + | + |
| **F** | 3-Methyl-1,3-butanediol | -0,34 | 75 % | 5 % | 20 % | + | + | + | + | + | + |
| **G** | 1,5-Pentandiol | -0,60 | 40 % | 5 % | 55 % | 0 | 0 | 0 | + | + | + |
| **H** | 1,3-Butandiol | -0,69 | 75 % | 5 % | 20 % | + | + | + | + | + | + |
| **I** | 1,2-Butandiol | -0,81 | 75 % | 5 % | 20 % | 0 | 0 | + | + | + | + |
| **J** | 2,3-Butandiol | -0,99 | 75 % | 5 % | 20 % | + | + | + | + | + | + |
| **K** | 1,3-Propandiol | -1,04 | 75 % | 5 % | 20 % | + | + | + | + | + | + |

| *Nicht erfindungsgemäße Vergleichsbeispiele* | | | | | | | | | | | |
|---|---|---|---|---|---|---|---|---|---|---|---|
| **L** | 1,2-Pentandiol | -0,28 | 95 % | 5 % | 0 % | 0 | 0 | 0 | 0 | 0 | + |
| **M** | 3-Methyl-1,3-butanediol | -0,34 | 95 % | 5 % | 0 % | 0 | 0 | 0 | 0 | 0 | 0 |
| **N** | 1,5-Pentandiol | -0,60 | 95 % | 5 % | 0 % | 0 | 0 | 0 | 0 | 0 | 0 |
| **O** | 1,3-Butandiol | -0,69 | 95 % | 5 % | 0 % | 0 | 0 | 0 | 0 | 0 | 0 |
| **P** | 1,2-Butandiol | -0,81 | 95 % | 5 % | 0 % | 0 | 0 | 0 | 0 | 0 | 0 |
| **Q** | 2,3-Butandiol | -0,99 | 95 % | 5 % | 0 % | 0 | 0 | 0 | 0 | 0 | 0 |
| **R** | 1,3-Propandiol | -1,04 | 95 % | 5 % | 0 % | 0 | 0 | 0 | 0 | 0 | 0 |
| **S** | 1,2-Propandiol | -1,34 | 95 % | 5 % | 0 % | 0 | + | + | + | + | + |
| **T** | - | - | 0 % | 5 % | 95 % | - | - | 0 | 0 | 0 | 0 |

Als Verteilungskoeffizienten (englisch: partition coefficient) "P(OW)" wird das Verhältnis der Konzentrationen einer Substanz im Gleichgewicht zwischen den zwei nicht mischbaren Flüssigkeiten n-Octanol und Wasser bezeichnet. Eine gängige Vergleichsgröße für das Phasenverhalten einer Substanz ist der dekadische Logarithmus des Verteilungskoeffizienten logP(OW) = log[c(n-Octanol)/c(Wasser)]. Dieser "logP"-Wert ist ein weithin verwendetes Maß für die Hydrophilie bzw. Lipophilie einer Substanz. Der Wert kann sowohl experimentell bestimmt, als auch berechnet werden. Der berechnete Wert wird auch als "calculated logP" ("ClogP") bezeichnet. In der Herstellung mehrphasiger Kosmetika ist der ClogP-Wert unter anderem nützlich zur Vorhersage über das Verteilungsverhalten von Inhaltsstoffen zwischen der Öl- und der Wasserphase. Hohe ClogP-Werte deuten darauf hin, dass die betreffende Substanz bevorzugt in die Ölphase übergeht, während niedrige und insbesondere negative ClogP-Werte auf eine hohe Wasserlöslichkeit hinweisen. Eine Vorhersage des Phasenverhaltens ist unter anderem nützlich, um die Aktivität eines Wirkstoffes bzw. dessen Transport an seinen Zielort zu optimieren. So sind zum Beispiel antimikrobielle Wirkstoffe dann am effektivsten, wenn sie sich in der Wasserphase oder an der Phasengrenze zu einer lipophilen Phase befinden.

Die erfindungsgemäßen Hexamidin-Salz-Lösungen sind innerhalb eines pH-Wert-Bereichs zwischen pH 3,0 und 6,0 chemisch stabil. Dies gilt insbesondere für eine Lagerung der Lösungen bei Temperaturen >20 °C. Der optimale pH-Bereich ist damit deutlich enger, als in der Literatur beschrieben. Die erhaltenen Resultate zur pH-Abhängigkeit der Stabilität von Hexamidin-Salzen sind in Tabelle 2 zusammengefasst. Während der Lagerung bei 42 °C erfolgt oberhalb pH 6,0 eine rasche Hydrolyse des Amidins innerhalb weniger Wochen (Tabelle 2, Lösungen J-N). Bei niedrigen pH-Werten < 3,0 wird ebenfalls eine allmähliche Hydrolyse des Amidins beobachtet (Tabelle 2, Lösungen A-C). Der pH-Wert von Hexamidin-Lösungen kann in Abhängigkeit vom Gegenion und von der Herstellungsmethode variieren. Daher muss der pH-Wert der Lösungen ggf. mit Hilfe einer Säure oder Base bzw. eines Puffersystems entsprechend angepasst werden.

**Tabelle 2: pH-Wert-abhängige Stabilität von Hexamidin-Diisethionat (HX) in Lösung**

| **Lsg.** | **Massen-%** | | | **pH-Regulator** | **pH (T0)** | **pH (10 Wo)** | **FI.-% Hexamidine (HPLC, 210 nm) nach X Wochen Lagerzeit bei 42 °C** | | | |
|---|---|---|---|---|---|---|---|---|---|---|
| | **1,2-Pentandiol** | **H₂O** | **HX** | | | | **T0** | **2 Wo** | **4 Wo** | **10 Wo** |
| **A** | 75% | 20% | 5% | Zitronensäure | 2,8 | 2,9 | 99,7% | 99,1% | 99,0% | **97,1%** |
| **B** | 75% | 20% | 5% | DL-Milchsäure | 2,8 | 2,9 | 99,7% | - | 98,8% | **97,2%** |
| **C** | 47,5% | 47,5% | 5% | Zitronensäure | 2,8 | 2,8 | 99,3% | 99,0% | 98,8% | **96,8%** |
| **D** | 47,5% | 47,5% | 5% | - | 4,1 | 4,3 | 99,3% | 99,1% | 99,3% | 99,2% |
| **E** | 75% | 20% | 5% | - | 4,3 | 4,9 | 99,8% | - | 99,1% | 99,1% |
| **F** | 47,5% | 47,5% | 5% | NaHCO₃ | 5,0 | 4,5 | 99,3% | 99,3% | 99,4% | 99,2% |
| **G** | 47,5% | 47,5% | 5% | NaHCO₃ | 5,5 | 4,9 | 99,3% | 99,3% | 99,2% | 99,2% |
| **H** | 75% | 20% | 5% | NaHCO₃ | 5,8 | 5,8 | 99,3% | 99,3% | 99,3% | 99,3% |
| **I** | 47,5% | 47,5% | 5% | NaHCO₃ | 5,8 | 5,7 | 99,4% | 99,3% | 99,3% | 99,2% |
| **J** | 47,5% | 47,5% | 5% | NaHCO₃ | 6,1 | 8,8 | 99,3% | 99,2% | **96,6%** | **65,4%** |
| **K** | 47,5% | 47,5% | 5% | NaHCO₃ | 6,5 | 8,7 | 99,3% | 99,2% | 99,1% | **88,3%** |
| **L** | 47,5% | 47,5% | 5% | NaHCO₃ | 7,0 | 8,8 | 99,4% | **93,1%** | **70,1%** | **44,7%** |
| **M** | 47,5% | 47,5% | 5% | NaHCO₃ | 7,4 | 8,7 | 99,2% | **85,1%** | **64,9%** | **41,4%** |
| **N** | 47,5% | 47,5% | 5% | NaHCO₃ | 7,7 | 8,8 | 99,3% | **84,3%** | **64,0%** | **41,2%** |

Als optionale Additiva zur Einstellung des pH-Wertes auf 3,0 bis 6,0 werden organische oder anorganische Basen ausgewählt, vorzugsweise Alkali- und Erdalkalioxide, -hydroxide,-alkoxide, -carbonate und -hydrogencarbonate oder organische oder anorganische Säuren, vorzugsweise organische Carbonsäuren und Sulfonsäuren. Besonders bevorzugt sind Isethionsäure und Citronensäure.

Die erfindungsgemäßen Lösungen sind in einem weiten Temperaturbereich klar, stabil und homogen. Eine Auskristallisation der festen Komponenten bei niedriger Temperatur tritt ebenso wenig auf, wie eine chemische Zersetzung des Hexamidins bei höherer Temperatur.

Kälte- und wärmestabile flüssige Mischungen wie die erfindungsgemäßen Hexamidin-Lösungen sind vorteilhaft, da zum einen die Entwicklung Schleimhaut reizender Stäube vermieden wird, welche bei der Handhabung fester Hexamidin-Salze auftreten. Weiterhin spart die Verwendung der Lösungen Zeit, Energie und Produktionskapazität, da flüssige Mischungen generell einfacher und genauer dosiert werden können, z.B. durch Zupumpen, Eingießen oder Einsaugen. Außerdem ist die Einarbeitung einer Flüssigkeit in flüssige Produkte schneller, effektiver und gleichmäßiger als die Einarbeitung eines Feststoffs. Beispielsweise wird eine Klumpenbildung vermieden.

Wie erwartet weisen die erfindungsgemäßen Lösungen eine im Vergleich zu den Einzelkomponenten erhöhte antimikrobielle Aktivität auf. Tabelle 3 zeigt exemplarisch die minimalen inhibitorischen Konzentrationen (MIC) der erfindungsgemäßen Lösung gemäß Tabelle 1C im Vergleich zu ihren Einzelkomponenten. Der Testablauf wird in Beispiel 2 beschrieben.

**Tabelle 3: Minimale Hemmkonzentrationen (MIC) gegenüber Mikroorganismen**

| **Mikroorganismus** | **Minimale inhibitorische Konzentrationen (MIC)** | | |
|---|---|---|---|
| | **Lösung gemäß Tabelle 1C (5 % Hexamidine Diisethionate)** | **Hexamidine Diisethionate** | **1,2-Pentandiol** |
| **Staphylococcus aureus** | 0,025 % | 0,0025 % | 4,0 % |
| **Pseudomonas aeruginosa** | 1,0 % | 0,1 % | 2,5 % |
| **Escherichia coli** | 0,5 % | 0,01 % | 2,5 % |
| **Aspergillus brasiliensis** | 0,05 % | 0,0005 % | 2,5 % |
| **Candida albicans** | 0,5 % | 0,01 % | 2,5 % |

Überraschend wurde jedoch gefunden, dass die erfindungsgemäßen Lösungen noch einen zusätzlichen synergistischen Effekt zeigen: Im Vergleich zum enthaltenden Alkandiol weisen sie eine erhöhte Fähigkeit zur Feuchthaltung der menschlichen Haut auf. So wurde beispielsweise gefunden, dass eine 2 %ige wässrige Lösung der erfindungsgemäßen Lösung aus Tabelle 1C bei Applikation auf der Haut einen stärkeren feuchthaltenden Effekt hat, als eine 3 %ige wässrige Lösung von 1,2-Pentandiol. Dies entspricht mindestens einer Verdopplung der Effektivität des Feuchthaltemittels 1,2-Pentandiol innerhalb der erfindungsgemäßen Lösung. Die mit Hilfe einer Corneometrie-Messung erhaltenen Daten sind in Tabelle 4 zusammengefasst. Die Durchführung des Tests wird in Beispiel 3 beschrieben.

**Tabelle 4: Corneometrie-Test zur feuchthaltenden Wirkung**

| **Testsubstanz** | **Konzentration** | **Corneometrie, % Veränderung im Vergleich zu T0** | | | | |
|---|---|---|---|---|---|---|
| | | **Nach 1 h** | **2 h** | **4 h** | **8 h** | **24 h** |
| **1,2-Pentandiol** | 3 % in H₂O | +18,8 % | +31,6 % | +37,7 % | +21,6 % | +13,1 % |
| **Lösung nach Tabelle 1C** | 2 % in H₂O (= 1,5 % 1,2-Pentandiol) | +29,6 % | +37,2 % | +26,1 % | +22,0 % | +18,5 % |
| **Glycerin** | 3 % in H₂O | +34,3 % | +40,2 % | +39,5 % | +29,9 % | +17,5 % |
| **Dest. H₂O** | 100 % | -6,1 % | -5,6 % | -2,9 % | -0,4 % | +1,6 % |

Die erfindungsgemäßen Lösungen können als Breitband-Konservierungsmittel in Kosmetika und Körperpflegeprodukten eingesetzt werden, wobei die Konzentration des Hexamidins im Endprodukt 0,1 Gew.-% nicht übersteigt. Dabei sind die erfindungsgemäßen Lösungen in der Lage, diverse Mikroorganismen abzutöten. Generell umfasst der Ausdruck "Mikroorganismus" Bakterien und Pilze, insbesondere gram-positive und gram-negative Bakterien, sowie Hefen und Schimmelpilze. Beispiele für Zielorganismen (ohne ausschließenden Charakter) sind: *Escherichia coli, Staphylococcus aureus, Enterococcus hirae, Pseudomonas aeruginosa, Burkholderia cepacia, Candida albicans* und/oder *Aspergillus brasiliensis.*

Als das mindestens eine Hexamidin-Salz a) werden Salze des Hexamidins eingesetzt, die für kosmetische oder dermatologische Anwendungen geeignet sind. Bevorzugt sind Salze des Hexamidins mit Carbonsäuren und/oder Sulfonsäuren und/oder Phenolen. Besonders bevorzugt wird Hexamidin als Salz der Isethionsäure eingesetzt.

Als Alkandiol-Komponente wird mindestens ein Alkandiol b) verwendet, dessen dekadischer Logarithmus des *n*-Oktanol-Wasser-Verteilungskoeffizienten (ClogP) zwischen -0,2 und -1,1 liegt. Vorzugsweise ist das mindestens eine Alkandiol b) ein C3- bis C5-Alkandiol, bevorzugt ein C4- bis C5-Alkandiol, besonders bevorzugt 1,2-Pentandiol.

Das im Produkt enthaltene mindestens eine Alkandiol b) kann zusätzliche Funktionen innerhalb des Fertigproduktes haben, z.B. als Feuchthaltemittel, als penetrationsförderndes Reagenz oder als Mittel zur Beeinflussung des Hautgefühls.
Zur Stabilisierung der erfindungsgemäßen Lösungen wird Wasser c) in einer Konzentration von 5 - 60 Gew.-% eingesetzt, bevorzugt in einer Konzentration von 5 - 30 Gew.-%.

Unabhängig von ihren antimikrobiellen und hautbefeuchtenden Eigenschaften können die in den erfindungsgemäßen Lösungen enthaltenen Komponenten auch andere Aufgaben erfüllen. Beispiele ohne ausschließenden Charakter sind Anwendungen als Konditionierer, Hautaufheller, Peeling-Mittel, Enzyminhibitor, Antioxidans, Licht- und UV-Schutz, zur Regulierung der Talgproduktion, zur Beeinflussung der Rheologie bzw. der Viskosität, sowie zur positiven Beeinflussung altersbedingter und/oder umweltbedingter Haut- bzw. Haar-Veränderungen.

Unter Verwendung der erfindungsgemäßen Lösung kann auch ein Produkt hergestellt werden, insbesondere ein kosmetisches und/oder pharmazeutisches und/oder dermatologisches und/oder hygienisches Produkt, enthaltend eine Lösung wie oben erläutert. In anderen Worten wird die erfindungsgemäße Lösung zur Konservierung kosmetischer und/oder pharmazeutischer und/oder dermatologischer und/oder hygienischer Produkte, insbesondere kosmetischer und dermatologischer Produkte, insbesondere zur Wachstumshemmung oder Abtötung von Mikroorganismen verwendet.

Eine weitere Verwendung der erfindungsgemäßen Lösung erreicht man bei Produkten, denen die erfindungsgemäße Lösung zum Zwecke einer Feuchthaltung der Haut zugesetzt wird. Als "hygienische Zubereitung" bzw. "hygienisches Produkt" werden insbesondere Haushalts- oder Reinigungsprodukte, sowie Riechstoffzubereitungen verstanden.

Die Zugabe der erfindungsgemäßen Lösungen zu dem kosmetischen und/oder pharmazeutischen und/oder dermatologischen und/oder hygienischen Produkt kann zu jedem beliebigen Zeitpunkt während der Produktion erfolgen, z.B. während der Herstellung einer wässrigen Phase oder am Ende des Produktionsablaufs.

Im Umfang der Erfindung ist ebenfalls ein Verfahren zur Wachstumshemmung oder Abtötung von Mikroorganismen enthalten, bei welchem die erfindungsgemäßen Lösungen zu Produkten aus den Anwendungsbereichen Kosmetik, Dermatologie, Körperpflege und Körperhygiene zugesetzt werden.

Weiterhin Umfang der Erfindung ist ein Verfahren zur Feuchthaltung der Haut, bei welchem die erfindungsgemäßen Lösungen zu diesem Zweck Anwendung finden.

Verwendung findet die erfindungsgemäße Lösung auch in einem Produkt, insbesondere einem kosmetischen oder dermatologischen Produkt, enthaltend die erfindungsgemäße Lösung, wobei das Produkt 0,1 - 2,0 Gew.-% Alkandiol(e) und 0,01 - 0,1 Gew.-% Amidin(e) enthält.

Das jeweilige Produkt kann in beliebiger Form vorliegen, insbesondere als:
a. Lösung,
b. Suspension,
c. Emulsion,
d. Gel,
e. Salbe,
f. Paste,
g. Pulver,
h. in Stücken oder als Block vorliegender Feststoff,
i. Schaum,
j. auf Mikroverkapselung, Liposomen oder ähnlichen mikroskopischen Strukturen basierendes Formulierungssystem.
k. Kombinationen der Formen a - j

### Beispiele / experimenteller Teil

### Beispiel 1 - Physikalische Stabilität der erfindungsgemäßen Lösungen

Hexamidin-Diisethionat wird bei 50 °C in verschiedenen Alkandiolen gelöst, ggf. unter Zusatz von Wasser. Die Lösungen werden während 16-20 h auf -20 °C temperiert. Anschließend werden die Mischungen innerhalb von 10 h in 2 °C-Schritten auf +20 °C erwärmt. Anschließend werden die Mischungen innerhalb von 10 h in 2 °C-Schritten wieder auf -20 °C abgekühlt. Die beobachteten Aggregatzustände der Mischungen sind in Tabelle 1 zusammengefasst (- = Feststoff, 0 = zweiphasige Mischung, + = klare Flüssigkeit).

### Beispiel 2 - Ermittlung der minimalen inhibitorischen Konzentration

Minimale inhibitorische Konzentrationen gegenüber Bakterien, Pilzen und Hefen wurden mit Hilfe eines Suspensionstests ermittelt. Dazu wurden neunstufige Verdünnungsreihen der jeweiligen Testsubstanzen in 1:1-Mischungen aus CASO-Bouillon und destilliertem Wasser hergestellt. Für die Tests mit Pilzen und Hefen wurde jeweils 2 % Dextrose zugesetzt. Jeder Mikroorganismus wurde separat in einem Reagenzglas getestet, das 10 mL des genannten Kulturmediums enthielt. Jedem Reagenzglas wurde 0,1 mL einer Suspension zugesetzt, die 5 x 10⁸ koloniebildende Einheiten (KBE) für Bakterien bzw. 1 x 10⁷ KBE für Pilze und Hefen enthielt. Jedes Reagenzglas wurde für 3 Tage bei 37 °C (Bakterien) bzw. für 4 Tage bei 30 °C (Pilze und Hefen) inkubiert. Als minimale inhibitorische Konzentration wurde diejenige Konzentration festgelegt, bei der nach Ablauf des Testzeitraums weder eine Trübung, noch sonstige sichtbare Zeichen eines Mikrobenwachstums beobachtet wurden. Die erhaltenen Resultate sind in Tabelle 3 aufgeführt.

### Beispiel 3 - Ermittlung der feuchthaltenden Wirkung mittels Corneometrie

Zur Untersuchung der feuchthaltenden Wirkung verschiedener Substanzen und Gemische wurden die Testsubstanzen mit destilliertem Wasser auf eine Konzentration von 2-3 % verdünnt. Die verdünnten Lösungen wurden auf die Vorderarme gesunder, freiwilliger Testpersonen aufgetragen (Anzahl: 12, Alter: 19-64 Jahre). Vor und nach der einfachen Applikation wurde die Hautfeuchte der behandelten Hautstellen instrumentell mit Hilfe eines Corneometers CM825, verbunden mit einem Cutometer dual MPA 580 der Firma Courage & Khazaka gemessen. Allen Messungen voran ging eine Akklimatisierung der Testpersonen in kontrollierter Umgebung (21 °C ± 1 °C, 45 % ± 10 % Luftfeuchtigkeit). Die Messergebnisse werden als prozentuale Veränderung gegenüber dem Ausgangwert nach 1 h, 2 h, 4 h, 8 h und 24 h angegeben und sind in Tabelle 4 zusammengefasst.

### Beispiel 4 - Duschgel, konserviert mit erfindungsgemäßer Lösung nach Tabelle 1C

| **Phase** | **Inhaltstoff** | **INCI-Name** | **%** |
|---|---|---|---|
| A | Wasser | *Aqua* | 72.4 |
| | Xanthan-Gummi | *Xanthan gum* | 0.6 |
| | Plantacare 818UP | *Cocoglucoside* | 15.0 |
| | Plantapon ACG HC | *Sodium cocoamphoacetate* | 5.0 |
| | Tegobetain F50 | *Cocamidopropyl betain* | 5.0 |
| B | Zitronensäure (50 % wässr. Lsg.) | *Citric acid (and) Aqua* | pH 5.5 |
| **C** | **Erfindungsgemäße Lösung nach Tabelle 1C** | ***Pentylene glycol (and) Water (and) Hexamidine Diisethionate*** | **2.0** |

Xanthan-Gummi und Wasser werden bei 700-800 U/min bis zur vollständigen Hydratisierung des Geliermittels gerührt. Die übrigen Komponenten der Phase A werden bei 400 U/min in der angegebenen Reihenfolge zugegeben. Der pH-Wert der Mischung wird nach Bedarf auf 5,5 abgesenkt. Anschließend wird die erfindungsgemäße Lösung unter Rühren zugegeben.

### Vergleichsbeispiel 5 - Duschgel, konserviert mit Hexamidin-Diisethionat

| **Phase** | **Inhaltstoff** | **INCI-Name** | **%** |
|---|---|---|---|
| A | Wasser | *Aqua* | 62.4 |
| | Xanthan-Gummi | *Xanthan gum* | 0.6 |
| | Plantacare 818UP | *Cocoglucoside* | 15.0 |
| | Plantapon ACG HC | *Sodium cocoamphoacetate* | 5.0 |
| | Tegobetain F50 | *Cocamidopropyl betain* | 5.0 |
| B | Zitronensäure (50 % wässr. Lsg.) | *Citric acid (and) Aqua* | pH 5.5 |
| C | Wasser | *Aqua* | 10.0 |
| | **MinaSolve Hexam** | ***Hexamidine Diisethionate*** | **0.1** |

Xanthan-Gummi und Wasser werden bei 700-800 U/min bis zur vollständigen Hydratisierung des Geliermittels gerührt. Die übrigen Komponenten der Phase A werden bei 400 U/min in der angegebenen Reihenfolge zugegeben. Der pH-Wert der Mischung wird nach Bedarf auf 5,5 abgesenkt. Anschließend wird Hexamidin-Diisethionat in Wasser gelöst und die erhaltene wässrige Lösung wird unter Rühren zugegeben.

### Vergleichsbeispiel 6 - Duschgel, konserviert mit Pentylene Glycol

| **Phase** | **Inhaltstoff** | **INCI-Name** | **%** |
|---|---|---|---|
| A | Wasser | *Aqua* | 71.4 |
| | Xanthan-Gummi | *Xanthan gum* | 0.6 |
| | Plantacare 818UP | *Cocoglucoside* | 15.0 |
| | Plantapon ACG HC | *Sodium cocoamphoacetate* | 5.0 |
| | Tegobetain F50 | *Cocamidopropyl betain* | 5.0 |
| B | Zitronensäure (50 % wässr. Lsg.) | *Citric acid (and) Aqua* | pH 5.5 |
| C | **MinaCare Pentiol Green+** | ***Pentylene Glycol*** | **3.0** |

Xanthan-Gummi und Wasser werden bei 700-800 U/min bis zur vollständigen Hydratisierung des Geliermittels gerührt. Die übrigen Komponenten der Phase A werden bei 400 U/min in der angegebenen Reihenfolge zugegeben. Der pH-Wert der Mischung wird nach Bedarf auf 5,5 abgesenkt. Anschließend wird Pentylene Glycol unter Rühren zugegeben.

### Beispiel 7 - O/W-Creme, konserviert mit erfindungsgemäßer Lösung nach Tabelle 1C

| **Phase** | **Inhaltstoff** | **INCI-Name** | **%** |
|---|---|---|---|
| A | Wasser | *Aqua* | 84.4 |
| | **Erfindungsgemäße Lösung nach Tabelle 1C** | ***Pentylene glycol (and) Water (and) Hexamidine Diisethionate*** | **1.0** |
| | Xanthan-Gummi | *Xanthan gum* | 0.5 |
| B | Emulgade PL 68/50 | *Cetearyl Glucoside (and) Cetearyl Alcohol* | 5.0 |
| | Lipex Shea | *Butyrospermum Parkii (Shea) Butter* | 3.0 |
| | Jojobaöl | *Simmondsia Chinensis (Jojoba) Seed Oil* | 3.0 |
| | Haselnussöl | *Corylus Avellana (Hazelnut) Seed Oil* | 3.0 |
| C | Bioxan T70 | *Tocopherol* | 0.1 |
| D | Zitronensäure (50 % wässr. Lsg.) | *Citric acid (and) Aqua* | pH 5.5 |

Die erfindungsgemäße Lösung nach Tabelle 1C wird unter Rühren mit Wasser vermischt, wobei eine klare Lösung entsteht. Xanthan-Gummi wird zugegeben und bei 700-800 U/min bis zur vollständigen Hydratisierung des Geliermittels mit einem Dispergierrührer gerührt. Anschließend wird Phase A unter Rühren auf 75-80 °C erwärmt. Die Komponenten der Phase B werden gemeinsam auf 80 °C erhitzt und dabei geschmolzen. Phase B wird bei 75-80 °C zur heißen Phase A gegeben. Die Mischung wird 3 min bei 10 000 U/min mittels Ultra-Turrax vermischt. Anschließend wird die Emulsion 30 min bei 1000 U/min mittels Dispergierrührer gerührt, sowie bei 700 U/min bis zum Erreichen von 20-25 °C. Bei < 40 °C wird Tocopherol unter Rühren zugegeben. Abschließend wird der pH-Wert der Mischung nach Bedarf auf 5,5 abgesenkt.

### Vergleichsbeispiel 8 - O/W-Creme, konserviert mit Hexamidin-Diisethionat

| **Phase** | **Inhaltstoff** | **INCI-Name** | **%** |
|---|---|---|---|
| A | Wasser | *Aqua* | 85.3 |
| | **MinaSolve Hexam** | ***Hexamidine Diisethionate*** | **0.1** |
| | Xanthan-Gummi | *Xanthan gum* | 0.5 |
| B | Emulgade PL 68/50 | *Cetearyl Glucoside (and) Cetearyl Alcohol* | 5.0 |
| | Lipex shea | *Butyrospermum Parkii (Shea) Butter* | 3.0 |
| | Jojobaöl | *Simmondsia Chinensis (Jojoba) Seed Oil* | 3.0 |
| | Haselnussöl | *Corylus Avellana (Hazelnut) Seed Oil* | 3.0 |
| C | Bioxan T70 | *Tocopherol* | 0.1 |
| D | Zitronensäure (50 % wässr. Lsg.) | *Citric acid (and) Aqua* | pH 5.5 |

Hexamidin-Diisethionat wird unter Rühren in Wasser gelöst. Xanthan-Gummi wird zugegeben und bei 700-800 U/min bis zur vollständigen Hydratisierung des Geliermittels mit einem Dispergierrührer gerührt. Anschließend wird Phase A unter Rühren auf 75-80 °C erwärmt. Die Komponenten der Phase B werden gemeinsam auf 80 °C erhitzt und dabei geschmolzen. Phase B wird bei 75-80 °C zur heißen Phase A gegeben. Die Mischung wird 3 min bei 10 000 U/min mittels Ultra-Turrax vermischt. Anschließend wird die Emulsion 30 min bei 1000 U/min mittels Dispergierrührer gerührt, sowie bei 700 U/min bis zum Erreichen von 20-25 °C. Bei < 40 °C wird Tocopherol unter Rühren zugegeben. Abschließend wird der pH-Wert der Mischung nach Bedarf auf 5,5 abgesenkt.

### Vergleichsbeispiel 9 - O/W-Creme, konserviert mit Pentylene Glycol

| **Phase** | **Inhaltstoff** | **INCI-Name** | **%** |
|---|---|---|---|
| **A** | Wasser | *Aqua* | 82.4 |
| | **MinaCare Pentiol Green+** | ***Pentylene Glycol*** | **3.0** |
| | Xanthan-Gummi | *Xanthan gum* | 0.5 |
| **B** | Emulgade PL 68/50 | *Cetearyl Glucoside (and) Cetearyl Alcohol* | 5.0 |
| | Lipex shea | *Butyrospermum Parkii (Shea) Butter* | 3.0 |
| | Jojobaöl | *Simmondsia Chinensis (Jojoba) Seed Oil* | 3.0 |
| | Haselnussöl | *Corylus Avellana (Hazelnut) Seed Oil* | 3.0 |
| **C** | Bioxan T70 | *Tocopherol* | 0.1 |
| **D** | Zitronensäure (50 % wässr. Lsg.) | *Citric acid (and) Aqua* | pH 5.5 |

Pentylene Glycol wird unter Rühren in Wasser gelöst. Xanthan-Gummi wird zugegeben und bei 700-800 U/min bis zur vollständigen Hydratisierung des Geliermittels mit einem Dispergierrührer gerührt. Anschließend wird Phase A unter Rühren auf 75-80 °C erwärmt. Die Komponenten der Phase B werden gemeinsam auf 80 °C erhitzt und dabei geschmolzen. Phase B wird bei 75-80 °C zur heißen Phase A gegeben. Die Mischung wird 3 min bei 10 000 U/min mittels Ultra-Turrax vermischt. Anschließend wird die Emulsion 30 min bei 1000 U/min mittels Dispergierrührer gerührt, sowie bei 700 U/min bis zum Erreichen von 20-25 °C. Bei < 40 °C wird Tocopherol unter Rühren zugegeben. Abschließend wird der pH-Wert der Mischung nach Bedarf auf 5,5 abgesenkt.

### Beispiel 10 - Mikrobiologische Konservierunasmittelbelastunastests gemäß ISO 11930

Um die ausreichende antimikrobielle Konservierung der in den vorangehenden Beispielen und Vergleichsbeispielen erzeugten Körperpflegeprodukte zu überprüfen, wurden die Produkte jeweils einem mikrobiologischen Konservierungsmittelbelastungstest unterzogen. Die Prüfung besteht aus der Kontamination des Prüfproduktes mit einem durch die Norm ISO 11930 vorgegebenen Inokulum von fünf unterschiedlichen Arten von Mikroorganismen, der Entnahme von Proben aus dem Prüfprodukt nach 7, 14 und 28 Tagen und der Bestimmung der Anzahl von Testkeimen in den entnommenen Proben. Die konservierenden Eigenschaften sind ausreichend, wenn sich unter den Bedingungen der Prüfung eine eindeutige Verminderung oder ggf. keine Vermehrung der Keimzahl in den beimpften Prüfprodukt nach den vorgeschriebenen Zeiten ergibt. Die Akzeptanzkriterien gemäß ISO 11930 sind in Tabelle 5 zusammengefasst. Die angegebenen Werte entsprechen jeweils dem dekadischen Logarithmus der Änderung der Anzahl der koloniebildenden Einheiten je Gramm (Δ KBE/g). Positive Zahlen repräsentieren eine Reduktion der Keimzahl, negative Zahlen einen Anstieg. Die Änderungen der Keimbelastung der geprüften Produkte sind in Tabelle 6 und 7 protokolliert.

**Tabelle 5: Akzeptanzkriterien "A" für ausreichende Konservierung gemäß ISO 11930**

| **Testkeim** | **Minimal erforderliche Δ log(KBE/g) gemäß ISO 11930 (NI = no increase, keine höhere Keimbelastung als beim Vorwert)** | | |
|---|---|---|---|
| | **T7** | **T14** | **T28** |
| **Aspergillus brasiliensis** | - | ≥ 0 + NI | ≥ 1 + NI |
| **Candida albicans** | ≥ 1 | ≥ 1 + NI | ≥ 1 + NI |
| **Pseudomonas aeruginosa** | ≥ 3 | ≥ 3 + NI | ≥ 3 +NI |
| **Staphylococcus aureus** | ≥ 3 | ≥ 3 + NI | ≥ 3 + NI |
| **Escherichia coli** | ≥ 3 | ≥ 3 + NI | ≥ 3 + NI |

**Tabelle 6: Konservierungsmittelbelastungstests von Duschgelen**

| | **Duschgel nach Beispiel 4, konserviert mit 2,0 % der Lösung Tabelle 1C** (0,1 % Hexamidin und 1,5 % Pentylene Glycol) | | | **Duschgel nach Vergleichsbeispiel 5, konserviert mit 0,1 % Hexamidin-Diisethionat** | | | **Duschgel nach Vergleichsbeispiel 6, konserviert mit 3,0 % Pentylene Glycol** | | |
|---|---|---|---|---|---|---|---|---|---|
| | **[Δ log(KBE/g)]** | | | **[Δ log(KBE/g)]** | | | **[Δ log(KBE/g)]** | | |
| **Testkeim** | **T7** | **T14** | **T28** | **T7** | **T14** | **T28** | **T7** | **T14** | **T28** |
| **Aspergillus brasiliensis** | - | >2,6 | >2,6 | - | >2,6 | >2,6 | - | 0,6 | ***0,3*** |
| **Candida albicans** | >3,3 | >3,3 | >3,3 | ***2,9*** | >3,3 | >3,3 | 2,5 | >2,9 | >2,9 |
| **Pseudomonas aeruginosa** | >4,8 | >4,8 | >4,8 | ***1,7*** | ***0,6*** | ***0,0*** | >4,2 | >4,2 | >4,2 |
| **Staphylococcus aureus** | >4,5 | >4,5 | >4,5 | ***2,1*** | 4,5 | ***-0,7*** | 4,5 | >4,5 | >4,5 |
| **Escherichia coli** | >4,7 | >4,7 | >4,7 | ***1,5*** | ***1,1*** | >4,7 | >4,4 | >4,4 | >4,4 |

Das mit 2 % der erfindungsgemäßen Lösung nach Tabelle 1C konservierte Duschgel (Beispiel 4) erfüllt die A-Kriterien für ausreichende Konservierung kosmetischer Produkte gemäß ISO 11930, während die mit 0,1 % Hexamidin-Diisethionat (Vergleichsbeispiel 5) bzw. 3.0 % Pentylene Glycol (Vergleichsbeispiel 6) versetzten Gele die Kriterien nicht vollständig erfüllen. Die Abweichungen von der Norm sind jeweils fettgedruckt hervorgehoben.

**Tabelle 7: Konservierungsmittelbelastungstests von O/W-Emulsionen**

| | **Emulsion nach Beispiel 7, konserviert mit 1,0 % der Lösung Tabelle 1C** (0,05 % Hexamidin und 0,75 % Pentylene Glycol) | | | **Emulsion nach Vergleichsbeispiel 8, konserviert mit 0,1 % Hexamidin-Diisethionat** | | | **Emulsion nach Vergleichsbeispiel 9, konserviert mit 3,0 % Pentylene Glycol** | | |
|---|---|---|---|---|---|---|---|---|---|
| | **[Δ log(KBE/g)]** | | | **[Δ log(KBE/g)]** | | | **[Δ log(KBE/g)]** | | |
| **Testkeim** | **T7** | **T14** | **T28** | **T7** | **T14** | **T28** | **T7** | **T14** | **T28** |
| **Aspergillus brasiliensis** | - | >2,6 | >2,6 | - | >2,6 | >2,6 | - | 0,3 | ***0,1*** |
| **Candida albicans** | >3,3 | >3,3 | >3,3 | >3,3 | >3,3 | >3,3 | 3,2 | >4,9 | >4,9 |
| **Pseudomonas aeruginosa** | >4,8 | >4,8 | >4,8 | >4,8 | >4,8 | >4,8 | >4,6 | >4,6 | >4,6 |
| **Staphylococcus aureus** | >4,5 | >4,5 | >4,5 | >4,5 | >4,5 | >4,5 | ***1,1*** | ***2,6*** | >4,8 |
| **Escherichia coli** | >4,7 | >4,7 | >4,7 | >4,7 | >4,7 | >4,7 | ***1,6*** | >4,8 | >4,8 |

Die mit 1,0 % der erfindungsgemäßen Lösung nach Tabelle 1C konservierte O/W-Emulsion erfüllt die A-Kriterien für ausreichende Konservierung kosmetischer Produkte gemäß ISO 11930. Zur Erzielung der gleichen antimikrobiellen Wirkung ist die doppelte Menge von 0,1 % der Einzelsubstanz Hexamidin-Diisethionat erforderlich (Vergleichsbeispiel 8). Im Vergleich dazu sind selbst 3,0 % Pentylene Glycol (Vergleichsbeispiel 9) nicht ausreichend für eine vollständige Konservierung gemäß ISO 11930. Die Abweichungen von der Norm sind fettgedruckt hervorgehoben.

## Patentansprüche

1. Temperaturstabile Lösung bestehend aus
a) 0,1 - 10 Gew.-% mindestens eines Hexamidin-Salzes,
b) 40 - 95 Gew.-% eines Alkandiols mit einer C3- bis C5-Kohlenstoffkette und einem ClogP von -0,2 bis -1,1,
c) 5 - 60 Gew.-% Wasser
und
d) einem Additiv oder mehreren Additiva zur Einstellung des pH-Wertes der Lösung auf 3,0 bis einschließlich 6,0,
wobei die Gesamtmenge der Komponenten a) bis d) 100 Gew.-% beträgt.

2. Temperaturstabile Lösung nach Anspruch 1, **dadurch gekennzeichnet, dass** das Hexamidin-Salz a) Hexamidin-Diisethionat ist.

3. Temperaturstabile Lösung nach mindestens einem der Ansprüche 1 und 2, **dadurch gekennzeichnet, dass** das Alkandiol b) ausgewählt ist unter 1,3-Propandiol, 1,2-Butandiol, 1,3-Butandiol (Butylene Glycol), 2,3-Butandiol, 1,2-Pentandiol (Pentylene Glycol), 1,5-Pentandiol und 3-Methyl-1,3-butandiol (Isopentyldiol), vorzugweise 1,2-Pentandiol und 1,3-Butandiol, insbesondere bevorzugt 1,2-Pentandiol.

4. Temperaturstabile Lösung nach mindestens einem der Ansprüche 1 bis 3, **dadurch gekennzeichnet, dass** die optionalen Additiva d) ausgewählt sind unter organischen und anorganischen Basen, vorzugweise Alkali- und Erdalkalioxiden, -hydroxiden, -alkoxiden, -carbonaten, und -hydrogencarbonaten.

5. Temperaturstabile Lösung nach mindestens einem der Ansprüche 1 bis 3, **dadurch gekennzeichnet, dass** die optionalen Additiva d) ausgewählt sind unter organischen und anorganischen Säuren, vorzugsweise organischen Carbonsäuren und Sulfonsäuren, insbesondere bevorzugt unter Isethionsäure und Citronensäure.

6. Verwendung einer temperaturstabilen Lösung nach Anspruch 1 bis 5 zur Konservierung kosmetischer und dermatologischer Erzeugnisse und Wachstumshemmung bzw. Abtötung von Mikroorganismen.

7. Verwendung einer Lösung nach Anspruch 6 durch Zugabe zu Erzeugnissen aus Kosmetik, Dermatologie, Körperpflege und Körperhygiene.

8. Nicht-therapeutische Verwendung einer temperaturstabilen Lösung nach Anspruch 1 bis 5 zur Befeuchtung der Haut von Säugetieren.

9. Verwendung einer temperaturstabilen Lösung nach Anspruch 1 bis 5 durch Einbringung in ein kosmetisches oder dermatologisches Erzeugnis.

## Claims

1. Temperature-stable solution consisting of
a) 0.1 - 10% by weight of at least one hexamidine salt,
b) 40 - 95% by weight of an alkanediol having a C3 to C5 carbon chain and a ClogP of -0.2 to -1.1,
c) 5 - 60% by weight of water
and
d) one additive or more additives for adjusting the pH value of the solution from 3.0 to 6.0 inclusive,
wherein the total amount of components a) to d) is 100% by weight.

2. Temperature-stable solution according to claim 1, **characterized in that** the hexamidine salt a) is hexamidine diisethionate.

3. Temperature-stable solution according to at least one of claims 1 and 2, **characterized in that** the alkanediol b) is selected from 1,3-propanediol, 1,2-butanediol, 1,3-butanediol (butylene glycol), 2,3-butanediol, 1,2-pentanediol (pentylene glycol), 1,5-pentanediol and 3-methyl-1,3-butanediol (isopentyldiol), preferably 1,2-pentanediol and 1,3-butanediol, more preferably 1,2-pentanediol.

4. Temperature-stable solution according to at least one of claims 1 to 3, **characterized in that** the optional additives d) are selected from organic and inorganic bases, preferably oxides, hydroxides, alkoxides, carbonates, and hydrogen carbonates of alkali and alkaline earth metals.

5. Temperature-stable solution according to at least one of claims 1 to 3, **characterized in that** the optional additives d) are selected from organic and inorganic acids, preferably organic carboxylic acids and sulfonic acids, more preferably from isethionic acid and citric acid.

6. Use of a temperature-stable solution according to claim 1 to 5 for the preservation of cosmetic and dermatological products and inhibition of growth or killing of microorganisms.

7. Use of a solution according to claim 6 by adding to cosmetics, dermatology, personal care and personal hygiene products.

8. Non-therapeutic use of a temperature-stable solution according to claim 1 to 5 for moistening the skin of mammals.

9. Use of a temperature-stable solution according to claim 1 to 5 by incorporation into a cosmetic or dermatological product.

## Revendications

1. Solution stable en température constituée de
a) 0,1 - 10% en poids d'au moins un sel d'hexamidine,
b) 40 - 95% en poids d'un alcanediol ayant une chaîne carbonée en C3 à C5 et un ClogP de -0,2 à -1,1,
c) 5 - 60% en poids d'eau
et
d) un ou plusieurs additifs pour ajuster la valeur du pH de la solution de 3,0 à 6,0 inclus,
dans laquelle la quantité totale des composants a) à d) est de 100% en poids.

2. Solution stable en température selon la revendication 1, **caractérisée en ce que** le sel d'hexamidine a) est le diiséthionate d'hexamidine.

3. Solution stable en température selon au moins l'une quelconque des revendications 1 et 2, **caractérisée en ce que** l'alcanediol b) est choisi parmi le 1,3-propanediol, le 1,2-butanediol, le 1,3-butanediol (butylène glycol), le 2,3-butanediol, le 1,2-pentanediol (pentylène glycol), le 1,5-pentanediol et le 3-méthyl-1,3-butanediol (isopentyldiol), de préférence le 1,2-pentanediol et le 1,3-butanediol, plus de préférence le 1,2-pentanediol.

4. Solution stable en température selon au moins l'une quelconque des revendications 1 à 3, **caractérisée en ce que** les éventuels additifs d) sont choisis parmi les bases organiques et inorganiques, de préférence les oxydes, les hydroxydes, les alkoxydes, les carbonates et les hydrogénocarbonates de métaux alcalins et alcalino-terreux.

5. Solution stable en température selon au moins l'une quelconque des revendications 1 à 3, **caractérisée en ce que** les éventuels additifs d) sont choisis parmi les acides organiques et inorganiques, de préférence les acides organiques carboxyliques et les acides sulfoniques, plus préférentiellement parmi l'acide iséthionique et l'acide citrique.

6. Utilisation d'une solution stable en température selon les revendications 1 à 5 pour la conservation de produits cosmétiques et dermatologiques et l'inhibition de la croissance ou de la destruction de micro-organismes.

7. Utilisation d'une solution selon la revendication 6 en ajoutant à des produits cosmétiques, dermatologiques, de soins corporels et d'hygiène corporelle.

8. Utilisation non thérapeutique d'une solution stable en température selon la revendication 1 à 5 pour humidifier la peau de mammifères.

9. Utilisation d'une solution stable en température selon la revendication 1 à 5 par incorporation dans un produit cosmétique ou dermatologique.
